Europäisches Patentamt

(19) European Patent Office

Office européen des brevets

(11) Publication number: **0 190 350**
**A1**

# EUROPEAN PATENT APPLICATION
published in accordance with Art. 158(3) EPC

(21) Application number: 84902946.7

(22) Date of filing: 31.07.84

Data of the international application taken as a basis:

(86) International application number:
PCT/JP84/00387

(87) International publication number:
WO86/00924 (13.02.86 86/04)

(51) Int. Cl.⁴: **C 12 N 9/02**
**C 12 Q 1/26**

(43) Date of publication of application:
13.08.86  Bulletin  86/33

(84) Designated Contracting States:
DE FR GB

(71) Applicant: NAGAI, Yoshiki
Room 402, Nakamurabashi Condo, 5-17, Nukui 2-chome
Nerima-ku, Tokyo 176(JP)

(72) Inventor: NAGAI, Yoshiki
Room 402, Nakamurabashi Condo, 5-17, Nukui 2-chome
Nerima-ku, Tokyo 176(JP)

(74) Representative: Fisher, Bernard et al,
Raworth, Moss & Cook 36 Sydenham Road
Croydon Surrey CR0 2EF(GB)

(54) PROCESS FOR PREPARING HUMAN PROLINE HYDROXYLASE.

(57) A process for purifying human proline hydroxylase of human placenta origin, which comprises using electrophoresis and subsequent electric extraction in addition to conventional purification steps. Since human placenta to be used as starting material is easily available in a large amount, purified human proline hydroxylase can be obtained easily in a large amount with about the same yield as in the prior art. An antiserum specific to the purified enzyme is also prepared, radioimmunoassay according to two-antibody method being established. Measurement of the concentration of proline hydroxylase in human blood serum by the assay is useful for diagnosis of acute hepatitis and evaluation of the effect of embolism treatment for primary cancer of the liver.

Figure 2

A Method of Preparing Human Prolyl Hydroxylase

## Technical Field

This invention relates to a method of preparing human prolyl hydroxylase (EC 1. 14. 11. 2), an enzyme which plays an important role in the biosynthesis of collagen.

## Background Art

Prolyl hydroxylase, an intercellularly-located enzyme in animals and plants, plays an important role in the biosynthesis of collagen (Cardinale, G.J. et al (1974) Adv Enzymol 41:245-300). It was Stein et al who first reported the clinical significance of this enzyme in human serum (Stein, H.D. et al (1970) Lancet I, 106-109). They measured the activity of this enzyme in human serum essentially utilizing an assay procedure developed by Hutton et al (Hutton, J.J. et al (1966) Anal Biochem 16:384-394), but it is very difficult to use this procedure clinically, because it is so laborious and troublesome that large numbers of samples could not be handled in an assay and because a number of investigators failed to detect the activity of this enzyme in serum with this procedure, probably due to the presence of inhibitors in the serum (e.g. Takeuchi et al (Takeuchi, T. et al (1966) Gastroenterology 56:744-750)).

As an alternative to the above, an attempt to measure the concentration of prolyl hydroxylase in serum employing a radioimmunoassay system - convenient for measuring large numbers of samples in an assay - was successfully made by Tuderman et al (Tuderman, L. et al (1975) Eur J. Biochem 60:399-405). Before them, Kuutti et al had developed a procedure for purifying the enzyme from human fetal tissue. (Kuutti, E-R et al (1975) Eur J. Biochem 57:181-188), and Tuderman et al established the above radioimmunoassay utilizing the enzyme purified from human fetal tissue by the method of Kuutti et al and its specific antiserum.

In practice, however, it is also very difficult to carry out this radioimmunoassay, because it is not easy to obtain human fetal tissue. It is necessary to prepare the enzyme from human tissue in order to develop a radioimmunoassay system to measure the concentration of this enzyme in human serum, but no attempt has been made to purify this enzyme from any human tissue other than fetal tissue. Furthermore, the clinical significance of determinations of the concentration of this enzyme in human serum has been essentially unknown.

In accordance with the present invention, I have established a procedure for purifying this enzyme from human placental tissue - a material obtainable in large amounts with greater ease than human fetal tissue - and have demonstrated that measuring this enzyme's concentration in human serum with the newly-developed radioimmunoassay is useful in diagnosing acute hepatitis and in judging the effectiveness of transcatheter arterial embolization (TAE) with patients having hepatocellular carcinoma.

The purification of prolyl hydroxylase utilizing human placental tissue as a raw material can be carried out by adding gel electrophoresis and electrophoretic extraction to the previous procedure described by Kuutti et al. The initial steps of the purification such as homogenization, ammonium sulfate fractionation, affinity chromatography on poly-L-proline derivatized Sepharose 4B and gel chromatography on a Bio Gel A-1.5m column were almost the same as described by Kuutti et al. However, the enzyme solution thus obtained still contained impurities when examined by gel electrophoresis, and the following steps were necessary to isolate the enzyme to homogeneity. The enzyme solution obtained was electrophoresed on polyacrylamide gels according to the method of Davis (Davis, B.J. (1964) Ann NY Acad Sci 121:404-427). After the electrophoresis the proteins on the gels were stained with anilinonaphthalene sulfonate and the protein band corresponding to the band previously ascertained to have the activity of the enzyme was cut out under ultraviolet light (Hartman, B.K. et al (1969) Anal Biochem 30:391-394). The enzyme was electrophoretically extracted from these gel slices according to the method of Hashizume et al (Hashizume, S. et al (1984) Electrophoresis 5:30-34) and thus the enzyme was purified. With respect to the recovery, there was little difference between the present purification and that described by Kuutti et al.

The advantages of the present invention over the known prior art are as follows:

1)  A procedure is established for purifying prolyl hydroxylase from human placental tissue with additional steps.

2)  This invention enables one to prepare the enzyme in large quantities, because human placental tissue can be obtained in large amounts relatively easily.

3)  The inventor has demonstrated that to establish a radioimmunoassay system utilizing the enzyme thus purified and its specific antiserum prepared in animals (e.g. rabbits) and to measure the concentration of this enzyme in human serum with this radioimmunoassay is clinically useful in diagnosing acute hepatitis and in judging the effectiveness of TAE with patients having hepatocellular carcinoma.

## Brief Description of the Drawings

Figure 1A shows a polyacrylamide gel of the enzyme partially purified by the procedure described by Kuutti et al. A minor band is seen below the major band.

Figure 1B shows a polyacrylamide gel of the enzyme purified by adding gel electrophoresis and electrophoretic extraction to the procedure described by Kuutti et al. A and B were stained by Coomasie brilliant blue.

Figure 2 shows a single precipitin line between the enzyme and its antiserum on double immunodiffusion. a) Crude enzyme at the ammonium sulfate step. b) Purified prolyl hydroxylase. c) Rabbit antiserum against the purified enzyme.

Figure 3 shows the concentrations of serum immunoreactive prolyl hydroxylase (S-IRPH) in normal subjects and in patients with various diseases.

Figure 4 shows a comparison of the values of S-IRPH before and after TAE with patients having hepatocellular carcinoma.

Figure 5 shows the behaviour of S-IRPH values in a patient with hepatocellular carcinoma who received TAE repeatedly.

Best Mode for Carrying Out the Invention

I would like to explain the invention by presenting a concrete example:

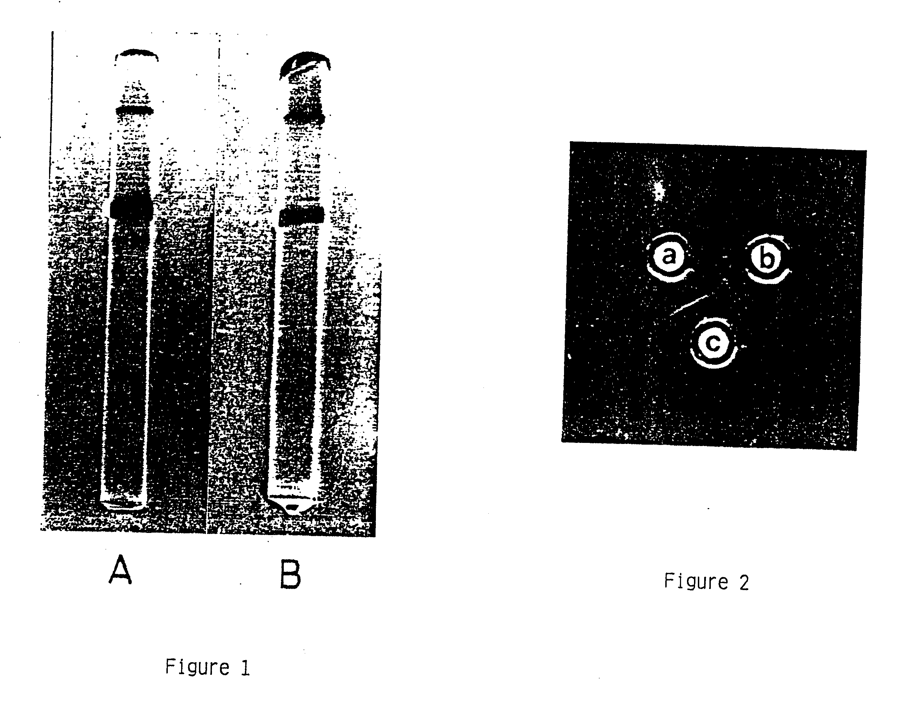

All procedures were carried out at 0-4°C. Human placental tissue (10.7 Kg) was homogenized in a Waring blender with a solution of 10 mM Tris HCl pH 7.8 containing 0.1 M NaCl, 0.1 M glycine, 0.1% Triton X-100 and 10 μM dithiothreitol, using 1 ml solution/g of tissue. The homogenate was centrifuged at 15,000 x g for 30 min and solid ammonium sulfate was slowly stirred into the supernatant fraction (14 ℓ). The protein which precipitated in the fraction of 20-70% saturation of ammonium sulfate was dissolved in a solution (1.7 ℓ) of 10 mM Tris HCl pH 7.8 containing 0.1 M NaCl, 0.1 M glycine and 10 μM dithiothreitol. The sample was dialyzed twice against 20 volumes of a solution containing the same components as above for 12 h. The dialyzate was centrifuged at 15,000 x g for 40 min and 82 ml of poly-L-proline derivatized Sepharose 4B (containing 2.6 mg of poly-L-proline with molecular weight of 40,000/1 ml gel volume) was added to the supernatant fraction. This mixture was stirred batchwise with a slowly rotating screw overnight so that the enzyme was adsorbed to the ligand. The reason why a batch method was used in this step was that in a column method the flow rate of the eluting fluid would be so low that adsorption could not be completed while the enzyme was still active. In this batch method, 80.5% of the enzyme activity was adsorbed onto the ligand. The poly-L-proline derivatized Sepharose 4B, which had adsorbed the enzyme, was collected on a glass filter and washed with 30 volumes of the same solution as described above. The washed gel was packed into a column (1.86 x 30 cm) and eluted with a solution made up of the same solution as described above containing 3 mg/ml of poly-L-proline having molecular weight of 9,000. The fractions having the enzyme activity were collected and concentrated by ultrafiltration in an Amicon ultrafiltration cell with PM-30 membrance down to 25.5 ml. The concentrate was centrifuged at 20,000 x g for 10 min to eliminate insoluble substances and the supernatant fraction was chromatographed on a Bio Gel A-1.5 m column (4.4 x 101 cm) and the effluent collected on a fraction collector. Enzyme activity and absorbance at 230 nm were determined for each fraction and those fractions having a high ratio of enzyme activity to absorbance at 230 nm were collected and concentrated by ultrafiltration in the above cell to 9.8 ml. When a portion of the concentrate was electrophoresed on a 7% polyacrylamide gel according to the method of Davis, there were two bands as shown in Fig. 1A. The majority of the enzyme activity was present in the major band, and the specific activity of the protein in the minor band was lower than that in the major band. Therefore, the above concentrate was electrophoresed on 7% polyacrylamide gels according to the method of Davis and the gels were stained with anilinonaphthalene sulfonate according to the method of Hartman et al. The protein band corresponding to the major band in Fig. 1A was cut out under ultraviolet light and the enzyme was electrophoretically extracted from the gel slices employing an apparatus of the type devised by Hashizume et al. In this way the enzyme was purified. When re-electrophoresed, the enzyme showed a single band (Fig. 1B). A summary of the purification is shown in Table I. With respect to the recovery, there was little difference between this purification and that described by Kuutti et al.

After gel electrophoresis and electrophoretic extraction in this purification, the enzyme activity and its specific activity decreased to 12% and 50% respectively of the values before these steps, but these steps were indispensable in purifying prolyl hydroxylase from human placental tissue.

Table I.  A summary of the purification of prolyl
hydroxylase from human placental tissue.

| Step | Protein ( mg ) | Activity ( Unit ) | Specific activity (Unit/mg) | Recovery ( % ) |
|---|---|---|---|---|
| 15,000 x g supernatant | 891,000 | 904,000 | 1.02 | 100 |
| Ammonium sulfate | 388,000 | 884,000 | 2.28 | 97:8 |
| Bio Gel A-1.5 m | 6.7 | 102,000 | 15,300 | 11.3 |
| Electrophoresis-electroextraction | 1.6 | 12,300 | 7,700 | 1.4 |

The Way in Which the Invention Is Capable of Exploitation
in Industry

Specific antiserum against the purified prolyl hydroxylase was prepared by immunizing rabbits.  Using Ouchterlony's double immunodiffusion technique only a single precipitin line was detected between the antiserum and the crude enzyme, which fused with the line of the purified enzyme (Fig. 2).  This result shows that the enzyme used for immunization was pure and the antiserum specific.  By using this specific antiserum, it is possible 1) to establish a radioimmunoassay system for measuring minute amounts of prolyl hydroxylase and 2) to localize the enzyme in tissue histochemically.

In the following paragraphs I would like to show that the development of a radioimmunoassay system using this specific antiserum and [125]I-labelled enzyme preparation and that the measurement of the concentration of prolyl hydroxylase in human serum with this radioimmunoassay system is useful in clinical medicine.

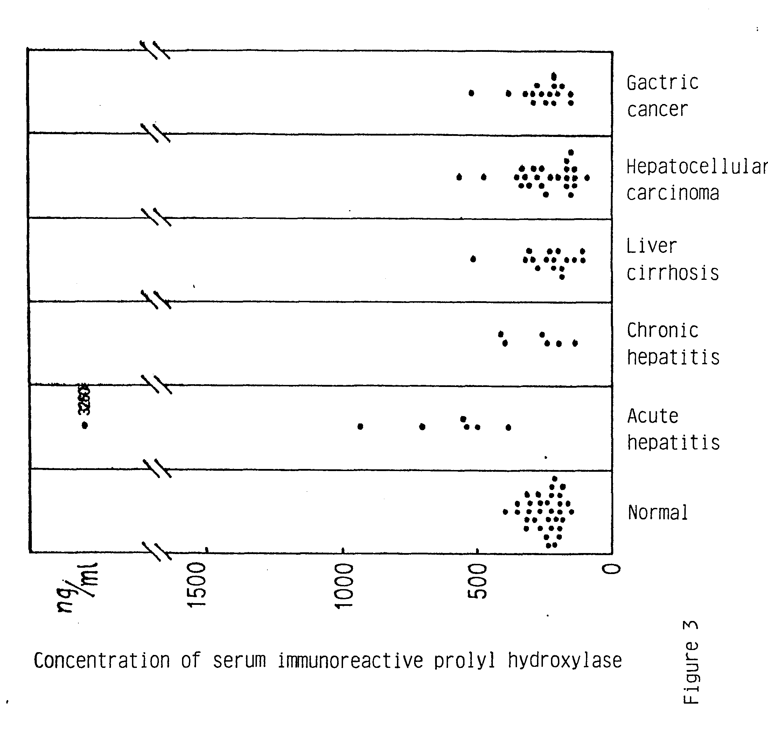

I established a radioimmunoassay based on a two-antibody system (Morgan, C.R. et al (1963) Diabetes 12:115-126) and measured the concentrations of S-IRPH in normal subjects and in patients with various diseases. The results are shown in Fig. 3. The mean value of the concentration of· S-IRPH in 33 normal subjects was 253 $\pm$ 59 (S.D.) ng/ml with a range of 155-400 ng/ml. The mean value of S-IRPH in 7 patients with acute hepatitis was 982 $\pm$ 1,020 (S.D.) ng/ml with a range of 385-3,260 ng/ml, which was significantly higher than that in normal subjects (P $<$ 0.001). But the mean values of S-IRPH in patients with chronic hepatitis, liver cirrhosis, hepatocellular carcinoma and gastric cancer showed no significant difference from that in normal subjects. The elevated values in acute hepatitis are considered to have resulted from the release of the enzyme from the damaged liver cells into the blood.

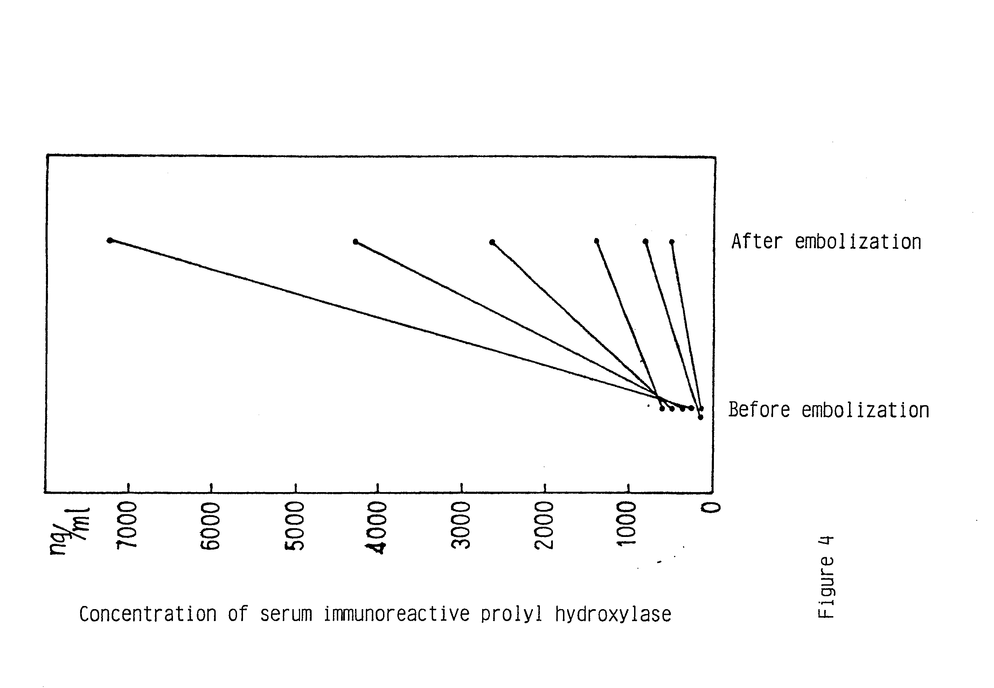

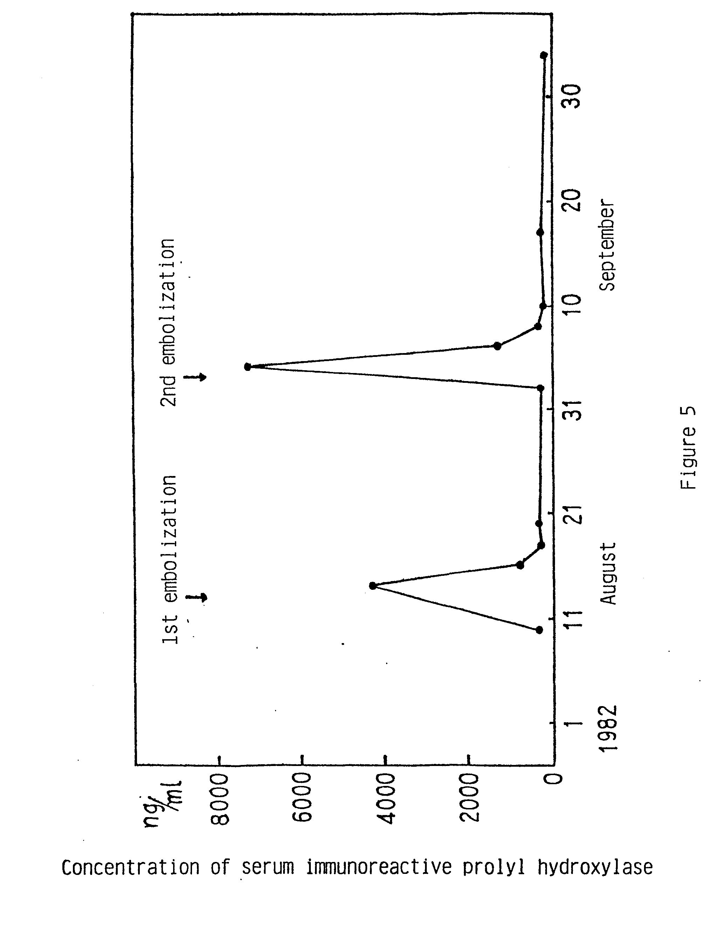

Recently, transcatheter arterial embolization therapy has been popularly performed on patients with unresectable hepatocellular carcinoma, in which an arterial catheter is superselectively inserted into the feeding artery of the tumor under fluorography and gelatin sponge particles (Spongel ®) are infused into the feeding artery through the catheter so that tumorous tissues are led to undergo ischaemic necrosis (Yamada, R. et al (1980) Osaka City Med J 26:81-96). When the values of S-IRPH before and after TAE were compared, they were found to have increased after TAE in all embolizations (Fig. 4). This effect is considered to be due to the release of the enzyme from the damaged tumorous tissue into the blood. The behaviour of S-IRPH values in a patient with hepatocellular carcinoma, who received TAE repeatedly, is shown in Fig.5, which also strongly suggests that the embolizations have led the hepatic tissues to undergo necrosis.

It will be apparent from the above observations that the determination of the concentration of S-IRPH with the newly developed radioimmunoassay is extremely valuable in diagnosing acute hepatitis and in judging the effectiveness of TAE with patients having hepatocellular carcinoma.

CLAIM:

A method of preparing pure human prolyl hydroxylase characterized by utilizing human placental tissue as a material.

Figure 2

Figure 1

Concentration of serum immunoreactive prolyl hydroxylase

Figure 3

Figure 4

0190350

4

Figure 5

Concentration of serum immunoreactive prolyl hydroxylase

# INTERNATIONAL SEARCH REPORT

**0190350**

International Application No. PCT/JP84/00387

## I. CLASSIFICATION OF SUBJECT MATTER (if several classification symbols apply, indicate all) [3]

According to International Patent Classification (IPC) or to both National Classification and IPC

Int. Cl$^3$  C12N 9/02, C12Q 1/26

## II. FIELDS SEARCHED

### Minimum Documentation Searched [4]

| Classification System | Classification Symbols |
|---|---|
| IPC | C12N 9/02, C12Q 1/26 |

Documentation Searched other than Minimum Documentation
to the Extent that such Documents are Included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| A | Chemical Abstracts. Vol. 83, No. 19 (1975. 11. 10), Kuutti, Eeva R. et. al. "Human prolyl hydroxylase. Purification, partial characterization, and preparation of antiserum to the enzyme." See pages 201 to 202, Abstract No. 159770m, Eur. J. Biochem. 1975, 57(1), 181-8 (Eng). | |
| A | Chemical Abstracts. Vol. 90, No. 23 (1979. 7. 4), Kuutti-Savolainen, Eeva R. et al. "Collagen biosynthesis enzymes in serum and hepatic tissue in liver disease. II. Galactosylhydroxylysyl glucosyltransferase. "See page 451, Abstract No. 184411x, Eur J. Clin. Invest. 1979, 9(1), 97-101 (Eng). | |

* Special categories of cited documents: [16]
"A" document defining the general state of the art which is not considered to be of particular relevance
"E" earlier document but published on or after the International filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

## IV. CERTIFICATION

| Date of the Actual Completion of the International Search [2] | Date of Mailing of this International Search Report [3] |
|---|---|
| October 20, 1984  (20. 10. 84) | November 12, 1984  (12. 11. 84) |
| International Searching Authority [1] | Signature of Authorized Officer [20] |
| Japanese Patent Office | |

Form PCT/ISA/210 (second sheet) (October 1981)